(19) Europäisches Patentamt
European Patent Office
Office européen des brevets

(11) EP 2 108 009 B1

(12) **EUROPÄISCHE PATENTSCHRIFT**

(45) Veröffentlichungstag und Bekanntmachung des Hinweises auf die Patenterteilung:
**01.06.2011 Patentblatt 2011/22**

(21) Anmeldenummer: **07848013.4**

(22) Anmeldetag: **10.12.2007**

(51) Int Cl.:
***C07C 45/75*** (2006.01) ***C07C 29/141*** (2006.01)
***C07C 47/19*** (2006.01) ***C07C 31/20*** (2006.01)
***C07C 31/22*** (2006.01) ***C07C 31/24*** (2006.01)

(86) Internationale Anmeldenummer:
**PCT/EP2007/063570**

(87) Internationale Veröffentlichungsnummer:
**WO 2008/080767 (10.07.2008 Gazette 2008/28)**

(54) **VERFAHREN ZUR HERSTELLUNG VON POLYALKOHOLEN AUS FORMALDEHYD MIT GERINGEM AMEISENSÄUREGEHALT**

PROCESS FOR PREPARING POLYALCOHOLS FROM FORMALDEHYDE WITH A LOW FORMIC ACID CONTENT

PROCÉDÉ DE FABRICATION DE POLYALCOOLS À PARTIR DE FORMALDÉHYDE AYANT UNE TENEUR EN ACIDE FORMIQUE RÉDUITE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **05.01.2007 EP 07100160**

(43) Veröffentlichungstag der Anmeldung:
**14.10.2009 Patentblatt 2009/42**

(73) Patentinhaber: **BASF SE**
**67056 Ludwigshafen (DE)**

(72) Erfinder:
- **SIRCH, Tilman**
  **67105 Schifferstadt (DE)**
- **STEINIGER, Michael**
  **67435 Neustadt (DE)**
- **MAAS, Steffen**
  **55270 Bubenheim (DE)**
- **RITTINGER, Stefan**
  **68199 Mannheim (DE)**
- **SCHLITTER, Stephan**
  **67117 Limburgerhof (DE)**
- **GUIXA GUARDIA, Maria**
  **68163 Mannheim (DE)**
- **SPENGEMAN, Todd, C**
  **Missouri City, TX 77459 (US)**
- **ANDRESS, Jeffrey, T.**
  **Lake Jackson, TX 77566 (US)**

(56) Entgegenhaltungen:
**WO-A-01/47853** **DE-A1- 1 957 591**
**DE-A1- 19 653 093**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 108 009 B1

## Beschreibung

Die Erfindung betrifft ein Verfahren zur Herstellung von Polymethylolverbindungen, allgemein auch Polyalkohole genannt, wie z. B. Neopentylglykol oder Trimethylolpropan.

Von den genannten Polyalkoholen werden beispielsweise Neopentylglykol("NPG") und Trimethyolpropan ("TMP") im Kunststoffbereich zur Herstellung von Lacken, Urethanen und Polyestern eingesetzt. Großtechnisch wird es zumeist nach dem Cannizzaro-Verfahren hergestellt. Um Trimethylolpropan nach diesem Verfahren herzustellen, wird n-Butyraldehyd mit einem Überschuss Formaldehyd in Gegenwart einer anorganischen Base umgesetzt. Dabei entsteht zugleich ein Äquivalent eines anorganischen Formiats als Koppelprodukt. Die Trennung des Salzes von Trimethylolpropan ist kompliziert und erfordert zusätzlichen Aufwand. Außerdem muss das anorganische Salz - falls es nutzbringend verwertet werden soll - aufgearbeitet und gereinigt werden und der Anfall des Koppelproduktes stellt einen Verlust der stöchiometrisch eingesetzten Mengen an Natronlauge und Formaldehyd dar. Zusätzlich sind die Ausbeuten bei dieser anorganischen Cannizzaro-Reaktion in Bezug auf den n-Butyraldehyd unbefriedigend, da im Verlauf der Reaktion hochsiedende Bestandteile gebildet werden, die nicht weiterverwertet werden können.

Ähnliche Probleme, wie für Trimethylolpropan geschildert, liegen bei der Herstellung von anderen Polyalkoholen wie Trimethylolethan (aus n-Propanal und Formaldehyd) oder Trimethylolbutan (aus n-Pentanal und Formaldehyd) oder Neopentylglykol (aus Isobutyraldehyd und Formaldehyd) vor. Zur Vermeidung dieser Nachteile wurde durch die WO 98/28253 ein Verfahren zur Herstellung von Polyalkoholen bekannt, bei dem Aldehyde mit 2 bis 24 C-Atomen in einer Aldolreaktion mit Formaldehyd unter Verwendung von tertiären Aminen als Katalysator zunächst zu den entsprechenden Alkanalen kondensiert und anschließend zu den entsprechenden Polyalkoholen hydriert werden (Hydrierverfahren). Dieses Verfahren ist koppelproduktarm. Nach der ersten Stufe werden nicht umgesetzte Aldehyde und ein Teil der Amin-Base im Allgemeinen vom Methylolalkanal destillativ abgetrennt und zurückgeführt. Im Destillationssumpf verbleiben neben den Produkten der Aldolisierung, den Methyoloalkanalen, wie zum Beispiel Hydroxypivalinaldehyd ("HPA"), Wasser, die Ameisensäuresalze der eingesetzten Base und Ameisensäure selbst. Das Methylolalkanal wird nach diesen Verfahren als 20 bis 70 Gew.-%ige wässrige Lösung erhalten. Das Dokument WO 01/47853 beschreibt ein Verfahren zur Herstellung von Polymethylolverbindungen wobei die Aldolreaktion mit einer wässrigen Formaldehydlösung eines Methanolgehalts von <0.3 Gew.-% durchgeführt wird.

Will man mehrwertige Alkohole wie Pentaerythrit, Neopentylglykol ("NPG")oder Trimethylolpropan ("TMP") aus wässrigen Methylolalkanal-Lösungen herstellen, müssen diese Lösungen hydriert werden.

Diese Hydrierung wird im allgemeinen bei Temperaturen von über 80°C durchgeführt. Es werden Rückspaltungen der Methylolgruppe zu freiem Aldehyd und darüber hinaus Ether-, Ester- und Acetalbildung im Hydrierreaktor beobachtet. Diese Nebenreaktionen führen zu niedrigen Hydrierselektivitäten und zu niedrigen Ausbeuten an mehrwertigem Alkohol.

Zudem sind viele Hydrierkatalysatoren unter diesen Bedingungen nicht stabil. Insbesondere Katalysatoren auf Basis der Oxide des Kupfers wie sie aus EP-A 44 444 und DE-A 19 57 591 bekannt sind, verlieren in Gegenwart dieser wässrigen Methylolalkanallösungen unter Hydrierbedingungen kontinuierlich an Hydrieraktivität , ihre Standzeit sinkt, und werden schlimmstenfalls unbrauchbar.

Es wurde erkannt, dass Ameisensäure, die herstellungsbedingt im Formaldehyd enthalten ist oder während der Aldolreaktion als Nebenprodukt über eine Cannizzaro-Reaktion aus Formaldehyd gebildet wurde, im Laufe der Hydrierung zu $CO_2$ und $H_2$ bzw. zu CO und $H_2O$ zersetzt wird. CO und $CO_2$ lassen sich im Abgas der Hydrierung nachweisen. Es wurde nun beobachtet, dass die Zersetzungsrate der Ameisensäure von der Temperatur und vom Alter des Katalysators abhängt.

CO und $CO_2$ erwiesen sich als Katalysatorgifte, die die Hydrieraktivität insbesondere von Kupferkatalysatoren negativ beeinflussen. Der gezielte Zusatz von CO oder $CO_2$ zum Wasserstoff führte zu einer signifikanten Abnahme der Hydrieraktivität des Kupferkatalysators, auch wenn der $H_2$-Partialdruck, die $H_2$-Absolutmenge und der pH-Wert im Hydrierreaktor konstant gehalten wurden.

Die verringerte Hydrieraktivität des Katalysators lässt sich zumindest zeitweise durch eine Erhöhung der Reaktionstemperatur kompensieren. Nachteilig ist jedoch, dass mit steigender Reaktionstemperaturen Nebenreaktionen zunehmen, die neben erhöhten Einsatzzahlen verunreinigtes Produkt zur Folge haben. So findet beispielsweise bei der Hydrierung von Hydroxypivalinaldehyd oder von Dimethylolbutanal zu den entsprechenden Alkoholen NPG und TMP mit zunehmender Temperatur eine Retroaldolreaktion statt. Die dabei entstehenden Aldehyde werden zu unerwünschten Nebenprodukten hydriert (bei NPG entstehen so Isobutanol und Methanol, bei TMP 2-Methylbutanol, 2-Ethyl-1,3-propandiol und Methanol) und die Ausbeute wird entsprechend vermindert. Im Falle der NPG-Synthese wird bei erhöhter Temperatur auch die Bildung des cyclischen Acetals von NPG und HPA verstärkt beobachtet. Dieses Nebenprodukt ist destillativ nicht von NPG zu trennen und führt daher zu einem unreineren Wertprodukt. Weiterhin fördern hohe Temperaturen die thermische Tischtschenko-Reaktion von HPA zu Hydroxypivalinsäureneopentylglykolester (HPN). Aufgrund dieser Nebenreaktionen ist die Temperaturerhöhung als Mittel zum Konstanthalten der Hydrieraktivität eines Katalysators durch wirtschaftliche Faktoren wie Ausbeute und Produktreinheit limitiert.

Der Erfindung lag daher die Aufgabe zugrunde, ein Verfahren zur Herstellung von Polymethylolverbindungen durch Kondensation von Aldehyden in einer Aldolreaktion mit Formaldehyd unter Verwendung von tertiären Aminen als Katalysator zu den entsprechenden Alkanalen und deren anschließender Hydrierung bereitzustellen, wie es beispielsweise durch die WO 98/28253 bekannt geworden ist, auf welche hier ausdrücklich Bezug genommen wird, bei dem die Rückspaltung von einmal gebildeten Methylolalkanalen weitgehend unterdrückt, die Bildung von Ethern, Estern und Acetalen weitgehend verhindert und ein positiver Effekt auf die Standzeit des Katalysators ausgeübt wird. Zudem sollte das Verfahren mehrwertige Alkohole mit guten Hydrierselektivitäten und Ausbeuten zugänglich machen.

Gelöst wird die Aufgabe durch ein Verfahren, bei dem die gewünschten Polymethylolverbindungen der Formel (I)

$$(HOCH_2)_2\text{-C-}R_2 \qquad (I)$$

in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet, durch Kondensation von Aldehyden mit 2 bis 24 C-Atomen in einer Aldolreaktion mit Formaldehyd unter Verwendung von tertiären Aminen als Katalysator zu Alkanalen der Formel (II)

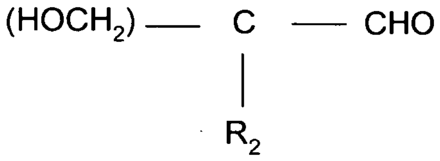 (II)

in der R jeweils unabhängig voneinander die oben genannte Bedeutung hat, hergestellt und anschließend hydriert werden, wobei die Aldolreaktion mit einer wässrigen Formaldehydlösung eines Ameisensäuregehalts von kleiner 150 ppm, vorzugsweise < 100 ppm, vorzugsweise < 50 ppm,durchgeführt wird.

Technisch verfügbarer Formaldehyd wird üblicherweise in wässriger Lösung in Konzentrationen von 30, 37 und 49 Gew.-% vertrieben. Dieser technische Formaldehyd enthält herstellungsbedingt durch Dehydrierung von Methanol Ameisensäure. Dieser Ameisensäurgehalt steigt bei der Lagerung technischen Formaldehyds weiter an. Durch den Einsatz einer wässrigen Formaldehydlösung mit erfindungsgemäß stark verringertem Ameisensäuregehalt wurde festgestellt, dass sich hohe Standzeiten der Hydrierkatalysatoren bei guter Ausbeute erreichen lassen.

Vorzugsweise wird ein Formaldehyd bzw. eine wässrige Formaldehydlösung eingesetzt, welche mit handelüblichen basischen Ionentauscher behandelt wurde. Als Anionentauscher kommen an sich bekannte, stark basische, schwach basische mittel basische gelförmige oder makroporöse Ionentauscher in Betracht. Dies sind zum Beispiel Anionentauscher der Struktur Polystyrolharz mit Divinylbenzol vernetzt mit tertiären Aminogruppen als funktionelle Gruppen. Auch Ionentauscher auf Basis Acrylsäure oder Methacrylsäure vernetzt mit Divinylbenzol oder Harze hergestellt durch Kondensation von Formaldehyd und Phenol kommen in Betracht.

Im einzelnen kommen zum Beispiel die Handelsprodukte Ambersep® 900, Amberlyst® und Amberlite® der Firma Rohm und Haas, Philadelphia, USA sowie Lewatit® der Firma Lanxess, Leverkusen in Betracht.

Bei der Durchführung des erfindungsgemäßen Verfahrens, das beispielhaft für die Herstellung von Neopentylglykol beschrieben werden soll, ohne jedoch darauf eingeschränkt zu werden, wird zunächst Isobutyraldehyd mit einer wässrigen Lösung von Formaldehyd eines Ameisensäuregehalts von kleiner 150 ppm, vorzugsweise < 100 ppm, vorzugsweise < 50 ppm, sowie einem Katalysator in Form eines tertiären Amins in einer Aldolreaktion umgesetzt. Dabei entsteht ein Gemisch aus Hydroxypivalinaldehyd, nicht umgesetzten Isobutyraldehyd und Formaldehyd sowie den genannten Amin-Katalysator und gegebenenfalls Wasser.

Das genannte Reaktionsgemisch wird anschließend einer Destillationsvorrichtung zugeführt, in der es in leichter und schwerer flüchtige Bestandteile aufgetrennt wird. Dabei werden die Destillationsbedingungen so gewählt, dass sich eine Fraktion aus Leichtsiedern bildet, in der als wesentliche Komponenten nicht umgesetzter Isobutyraldehyd, Formaldehyd, gegebenenfalls Wasser, ein Teil des Aminkatalysators enthalten sind. Diese sogenannte Leichtsiederfraktion wird wieder bei der Durchführung der Aldolreaktion, wie bereits beschrieben, eingesetzt.

Nach Abtrennung der Leichtsiederfraktion verbleibt bei der geschilderten destillativen Aufarbeitung ein schwerer flüchtiges Sumpfprodukt, das im wesentlichen aus Hydroxypivalinaldehyd und Wasser sowie einem Teil des Aminkatalysators besteht.

Durch den erfindungsgemäß verringerten Ameisensäuregehalt der wässrigen Formaldehydlösung ist eine stärkere Nebenproduktbildung an Ethern und Acetalen nicht zu befürchten.

Der so gewonnene Hydroxypivalinaldehyd wird dann in an sich bekannter Weise mit Wasserstoff katalytisch zu Neopentylglykol hydriert.

Alternativ zur vorstehend beschriebenen Arbeitsweise kann das erfindungsgemäße Verfahren auch so durchgeführt werden, dass das sich bei der Aldolreaktion ergebene Reaktionsgemisch einem Phasenscheider statt einer Destillationsvorrichtung zugeführt wird und in diesem Phasenscheider eine Auftrennung des Reaktionsgemisches in eine wässrige und eine organische Phase erfolgt. Dabei können die üblicherweise für flüssig-flüssig-Trennungen verwendeten Phasenscheidevorrichtungen, wie sie in Ullmanns Encyklopädie der technischen Chemie, 4. Auflage, Band 2, S. 560 - 565, Verlag Chemie, Weinheim, 1972 beschrieben sind, verwendet werden.

Die Aldolreaktion wird im allgemeinen bei einer Temperatur von 5 bis 100°C, bevorzugt von 15 bis 80°C durchgeführt und die Verweilzeit wird in Abhängigkeit von der Temperatur im allgemeinen auf 0,25 bis 12 Stunden eingestellt.

Bei der Aldolreaktion beträgt das Molverhältnis von jeweils frisch zugesetztem Isobutyraldehyd zu der hinzugefügten Menge Formaldehyd zweckmäßigerweise zwischen 1 : 2 und 1 : 5, bevorzugt 1 : 2 bis 1 : 3,5. Die Menge an bei der Aldolreaktion zugesetztem tertiärem Aminkatalysator beträgt in Bezug auf den zugesetzten Isobutyraldehyd in der Regel 0,001 bis 0,2, bevorzugt 0,01 bis 0,07 Äquivalente, d. h. das Amin wird in katalytischen Mengen eingesetzt.

Die sich anschließende Destillation zur Auftrennung in eine Leichtsiederfraktion und das Sumpfprodukt wird im allgemeinen bei 50 bis 200°C, vorzugsweise bei 90 bis 160°C und bei einem Druck von im allgemeinen 0,1 mbar bis 10 bar, vorzugsweise von 0,5 bis 5 bar, insbesondere bei Atmosphärendruck, durchgeführt.

Man erhält ein Aldolisierungsprodukt, das im wesentlichen aus Methylolalkanal wie zum Beispiel Hydroxypivalinaldehyd bzw. je nach eingesetzten Ausgangsverbindungen dem entsprechenden Alkanal besteht. Dieses dient als Hydrierfeed und wird in einem geeigneten Hydrierreaktor katalytisch hydriert.

Erfindungsgemäß verwendbare Katalysatoren sind für Hydrierungen geeignete Katalysatoren, die bevorzugt mindestens ein Metall der Nebengruppe 8 bis 12 des Periodensystems der Elemente wie Fe, Ru, Os, Co, Rh, Ir, Ni, Pd, Pt, Cu, Ag, An, Zn, Cd, Hg, bevorzugt Fe, Co, Ni, Cu, Ru, Pd, Pt, besonders bevorzugt Cu, bevorzugt auf einem üblichen Trägermaterial, besonders bevorzugt auf einem aus den Oxiden des Titans, Zirkoniums, Hafniums, Siliziums und/oder Aluminiums aufweisen. Die Herstellung der erfindungsgemäß verwendbaren Katalysatoren kann nach aus dem Stand der Technik bekannten Verfahren zur Herstellung derartigen Trägerkatalysatoren erfolgen. Bevorzugt verwendet werden können auch Trägerkatalysatoren, die Kupfer auf einem Aluminiumoxid- oder Titandioxid-haltigen Trägermaterial in An- oder Abwesenheit eines oder mehrer der Elemente Magnesium, Barium, Zink oder Chrom aufweist. Derartige Katalysatoren und ihre Herstellung sind aus WO 99/44974 bekannt.

Weiterhin sind kupferhaltige Trägerkatalysatoren wie sie z.B. in WO 95/32171 beschrieben sind und die in EP-A 44 444 und DE 19 57 591 offenbarten Katalysatoren für die erfindungsgemäße Hydrierung geeignet.

Die Hydrierung kann diskontinuierlich oder kontinuierlich z.B. in einem mit einer Katalysatorschüttung gefüllten Reaktorrohr durchgeführt werden, bei dem die Reaktionslösung über die Katalysatorschüttung z.B. in Riesel- oder Sumpffahrweise, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im allgemeinen zwischen 50 und 180°C, bevorzugt 90 und 140°C. Als Hydrierdruck werden im allgemeinen 10 bis 250 bar, bevorzugt 20 bis 120 bar angewandt.

Der Hydrierfeed wird vor dem Hydrierreaktoreingang mit tertiärem Amin vermischt bis der Hydrieraustrag einen pH-Wert von 7 bis 9 aufweist. Es ist auch möglich, den Hydrierfeed und das tertiäre Amin getrennt in den Reaktor einzuspeisen und dort zu vermischen.

Im übrigen können beliebige Hydriermethoden angewendet und Hydrierkatalysatoren eingesetzt werden, wie sie für die Hydrierung von Aldehyden üblich und in der Standardliteratur eingehend beschrieben sind.

Das so erhaltene Roh-Neopentylglykol kann in an sich üblicher Weise durch Destillation gereinigt werden.

Das erfindungsgemäße Verfahren kann mit oder ohne Zusatz von organischen Lösungsmitteln oder Lösungsvermittlern durchgeführt werden. Der Zusatz von Lösungsmitteln oder Lösungsvermittlern kann sich insbesondere bei der Verwendung langkettiger Aldehyde als Ausgangsmaterialien als vorteilhaft erweisen. Durch den Einsatz von Lösungsmitteln, die geeignete niedrigsiedende azeotrope Gemische mit den leichtsiedenden Verbindungen bei den einzelnen Destillationen des erfindungsgemäßen Verfahrens bildern, kann gegebenenfalls der Energieaufwand bei diesen Destillationen erniedrigt und/oder die destillative Abtrennung der Leichtsieder von den hochsiedenden Verbindungen erleichtert werden.

Als Lösungsmittel sind z. B. cyclische und acyclische Ether, wie THF, Dioxan, Methyl-tert.butylether oder Alkohole, wie Methanol, Ethanol oder 2-Ethylhexanol geeignet.

Die für die Aldolisierungsreaktion beschriebenen Reaktionsführungen können bei einem Druck von im allgemeinen 1 bis 30 bar, vorzugsweise 1 bis 15 bar, besonders bevorzugt 1 bis 5 bar, zweckmäßigerweise unter dem Eigendruck des betreffenden Reaktionssystems, durchgeführt werden.

Das neue Verfahren ist auf praktisch alle Alkanale mit einer Methylengruppe in $\alpha$-Stellung zur Carbonylgruppe anwendbar. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso können araliphatische Aldehyde als Ausgangsstoffe eingesetzt werden, vorausgesetzt, dass sie eine Methylengruppe in $\alpha$-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butylbutanal sowie entsprechende -n-pentanale, -n-hexanale, -n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -nhexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methylheptanal; 4-Methylpentanal, 4-Methylheptanal, 5-Methyl-hexanal, 5-Methylheptanal; 3,3,5-Trimethyl-n-pentyl-, 3,3-Diethylpentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4-Tetramethylpentylaldehyd; insbesondere $C_2$- bis $C_{12}$-n-Alkanale.

Neben dem bereits erwähnten Neopentylglykol das hier im wesentlichen und beispielhaft erläutert wurde, können vorzugsweise außerdem n-Butyraldehyd zur Herstellung von Trimethylolpropan, Acetaldehyd zur Herstellung von Pentaerythrit, Propionaldehyd zur Herstellung von Trimethylolethan und n-Pentanal zur Herstellung von Trimethylolbutan als Ausgangsverbindungen eingesetzt werden.

Als tert. Amine kommen bezüglich ihrer Eignung für die Kondensation von Aldehyden mit Formaldehyd an sich bekannte Amine, wie sie z. B. in DE-A 28 13 201 und DE-A 27 02 582 beschrieben sind, in Betracht. Besonders bevorzugt sind Tri-n-alkylamine, wie Triethylamin, Tri-n-propylamin, Tri-n-butylamin und insbesondere Trimethylamin.

Das erfindungsgemäße Verfahren zeichnet sich durch hohe Ausbeuten, bezogen sowohl auf den Ausgangsaldehyd als auch auf den Formaldehyd aus und führt zu sehr geringen Verlusten an Aminkatalysator.
Im folgenden soll die Erfindung anhand von Ausführungsbeispielen näher erläutert werden.

Beispiele

Bestimmung des Ameisensäuregehalts

Der Ameisensäuregehalt im Formaldehyd in ppm (parts per million) wurde acidimetrisch mit einer wässrigen NaOH-Lösung (0,01 mol/L) bestimmt. Für die Bestimmung wurde ein Metrohm TiNet-Titrationssystem mit Titrino 736GP mit Dosimat E685, eine Metrohm kombinierte Gaselektrode (6.0210.100) sowie ein Metrohm Temperaturfühler PT100/PT1000 verwendet. 46 g Formaldehyd in Form einer 49%igen wässrigen Lösung wurde bei Zimmertemperatur mit HPLC-Wasser auf 100 ml verdünnt. Die Probe wurde mit NaOH-Lösung dynamisch auf den Endpunkt (EP1) bei pH 6-6.5 titriert.
Die Berechnung des Ameisensäuregehalts erfolgte nach:

$$\frac{\text{Verbrauch (EP1) in ml (NaOH) x 460}}{\text{Einwaage in g}} = \text{ppm HCOOH}$$

Hydrierung von Hydroxypivalinaldehyd zu Neopentylglykol

Vergleichsbeispiel : Hydrierfeed A

a) Aldolisierung

1,1 mol Isobutyraldehyd wurden mit 1 mol Formaldehyd in Form einer 49 Gew.-%igen Lösung mit einem Gehalt an 1,5 Gew.-% Methanol und 200 ppm Ameisensäure und 4 mol.-% Trimethylamin, bezogen auf Isobutyraldehyd, bei 75°C 1 h lang gerührt. Die Reaktionslösung wurde eingeengt, indem bei Normaldruck Leichtsieder wie beispielsweise Isobutyraldehyd und ein Teil des Wassers abdestilliert wurden. Der erhaltene Sumpf bestand aus 75 Gew.-% Hydroxypivalinaldehyd, 20 Gew.-% Wasser und ca. 5 Gew.-% sonstigen organischen Nebenkomponenten.

Beispiel 1: Hydrierfeed B

Eine wässrige Formaldehydlösung mit einem Gehalt von 49 Gew.-% Formaldehyd, 1,5 Gew.-% Methanol und 200 ppm Ameisensäure wurde über den handelsüblichen, basischen Ionentauscher Ambersep® 900 OH der Firma Rohm und Haas Company, Philadelphia, USA geleitet. Nach dieser Behandlung wurde der Ameisensäuregehalt mittels Titration zu 10 ppm bestimmt.

Hydrierung von Hydrierfeed A und B

a) Katalysatoraktivierung

150 ml eines $Cu/Al_2O_3$-Katalysators gemäß Beispiel 1 der EP 44444 wurden in einem Rohrreaktor bei 190°C durch Überleiten eines Gemisches aus 5 Vol.-% Wasserstoff und 95 Vol.-% Stickstoff (Gesamtvolumen 50 Nl/h) drucklos 24 h aktiviert.

Hydrierung

Als Ausgangslösung diente das vorstehend als Hydrierfeed A beschriebene Gemisch. Der Hydrierfeed wird in Rieselfahrweise bei 37 bar $H_2$-Druck über den auf 105°C beheizten Reaktor gefahren. Die Belastung betrug 0,2 kg Hydroxypivylinaldehyd/ ($l_{Kat.}$*h). Im Hydrierzulauf wurde mit Trimethylamin in Form einer 15 gew.-%igen Lösung ein pH-Wert 7,9 im Hydrieraustrag eingestellt. Ein Teil des Hydrieraustrags wird dem Zulauf wieder zugemischt (Kreislauffahrweise). Über mehrere Tage wurde ein mittlerer Umsatz von 95,3% bei einem mittleren pH-Wert von 8,8 erzielt.

Anschließend wurde der Zulauf auf den erfindungsgemäßen Hydrierfeed B umgestellt. Der mittlere Umsatz bei dieser Einstellung betrug 95,9%.

**Patentansprüche**

1. Verfahren zur Herstellung von Polymethylolverbindungen der Formel (I)

$$(HOCH_2)_2\text{-C-}R_2 \qquad (I)$$

in der R jeweils unabhängig voneinander eine weitere Methylolgruppe oder eine Alkylgruppe mit 1 bis 22 C-Atomen oder eine Aryl- oder Aralkylgruppe mit 6 bis 22 C-Atomen bedeutet, durch Kondensation von Aldehyden mit 2 bis 24 C-Atomen in einer Aldolreaktion mit Formaldehyd unter Verwendung von tertiären Aminen als Katalysator zu Alkanalen der Formel (II)

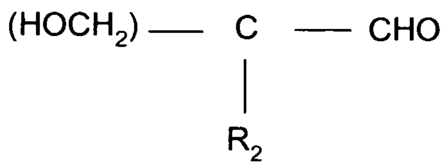

(II)

in der R jeweils unabhängig voneinander die oben genannte Bedeutung hat, und deren anschließende Hydrierung, **dadurch gekennzeichnet, dass** die Aldolreaktion mit einer wässrigen Formaldehydlösung eines Ameisensäuregehalts von < 150 ppm durchgeführt wird.

2. Verfahren nach Anspruch 1, **dadurch gekennzeichnet, dass** die Aldolreaktion mit einer wässrigen Formaldehydlösung eines Ameisensäuregehalts von < 100 ppm durchgeführt wird.

3. Verfahren nach einem der Ansprüche 1 oder 2, **dadurch gekennzeichnet, dass** Propionaldehyd zu Trimethylolethan, n-Butyraldehyd zu Trimethylolpropan, Acetaldehyd zu Pentaerythrit oder iso-Butyraldehyd zu Neopentylglykol umgesetzt wird.

4. Verfahren nach einem der Ansprüche 1 bis 3, **dadurch gekennzeichnet, dass** die Umsetzung kontinuierlich durchgeführt wird.

**5.** Verfahren nach einem der Ansprüche 1 bis 4, **dadurch gekennzeichnet, dass** als Katalysator tertiäre Amine in einer solchen Menge verwendet werden, dass sich im Reaktionsgemisch ein pH-Wert von 5 bis 12 einstellt.

**6.** Verfahren nach einem der Ansprüche 1 bis 5, **dadurch gekennzeichnet, dass** Trimethylamin als Katalysator verwendet wird.

## Claims

**1.** A process for preparing polymethylol compounds of the formula (I)

$$(HOCH_2)_2\text{-}C\text{-}R_2 \qquad (I)$$

where the radicals R are each, independently of one another, a further methylol group or an alkyl group having from 1 to 22 carbon atoms or an aryl or aralkyl group having from 6 to 22 carbon atoms, by condensation of aldehydes having from 2 to 24 carbon atoms with formaldehyde in an aldol reaction using tertiary amines as catalyst to form alkanals of the formula (II)

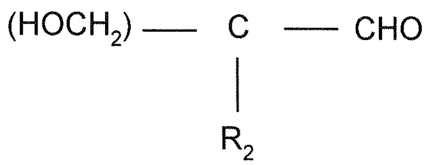

(II)

where the radicals R each independently have one of the abovementioned meanings, and subsequent hydrogenation of the latter wherein the aldol reaction is carried out using an aqueous formaldehyde solution having a formic acid content of < 150 ppm.

**2.** The process according to claim 1, wherein the aldol reaction is carried out using an aqueous formaldehyde solution having a formic acid content of < 100 ppm.

**3.** The process according to either claim 1 or 2, wherein propionaldehyde is converted into trimethylolethane, n-butyraldehyde is converted into trimethylolpropane, acetaldehyde is converted into pentaerythritol or isobutyraldehyde is converted into neopentyl glycol.

**4.** The process according to any of claims 1 to 3, wherein the reaction is carried out continuously.

**5.** The process according to any of claims 1 to 4, wherein tertiary amines are used as catalyst in such an amount that a pH of from 5 to 12 is established in the reaction mixture.

**6.** The process according to any of claims 1 to 5, wherein trimethylamine is used as catalyst.

## Revendications

**1.** Procédé pour la préparation de composés de type polyméthylol de formule (I)

$$(HOCH_2)_2\text{-}C\text{-}R_2 \qquad (I)$$

dans laquelle R représente chaque fois indépendamment un autre groupe méthylol ou un groupe alkyle ayant de 1 à 22 atomes de carbone ou un groupe aryle ou aralkyle ayant de 6 à 22 atomes de carbone, par condensation d'aldéhydes ayant de 2 à 24 atomes de carbone, dans une réaction d'aldol avec du formaldéhyde, avec utilisation d'amines tertiaires en tant que catalyseur, pour l'obtention d'alcanals de formule (II)

$$\begin{array}{l}(HOCH_2) - C - CHO \\ \qquad\qquad\;\; | \\ \qquad\qquad\; R_2\end{array} \qquad (II)$$

dans laquelle R a chaque fois indépendamment la signification donnée ci-dessus, et hydrogénation subséquente de ceux-ci, **caractérisé en ce que** la réaction d'aldol est effectuée avec une solution aqueuse de formaldéhyde ayant une teneur en acide formique de < 150 ppm.

**2.** Procédé selon la revendication 1, **caractérisé en ce que** la réaction d'aldol est effectuée avec une solution aqueuse de formaldéhyde ayant une teneur en acide formique de < 100 ppm.

**3.** Procédé selon la revendication 1 ou 2, **caractérisé en ce qu'**on convertit du propionaldéhyde en triméthyloléthane, du n-butyraldéhyde en triméthylolpropane, de l'acétaldéhyde en pentaérythritol ou de l'isobutyraldéhyde en néopentylglycol.

**4.** Procédé selon l'une quelconque des revendications 1 à 3, **caractérisé en ce que** la réaction est effectuée en continu.

**5.** Procédé selon l'une quelconque des revendications 1 à 4, **caractérisé en ce qu'**on utilise comme catalyseur des amines tertiaires en une quantité telle qu'un pH de 5 à 12 s'ajuste dans le mélange réactionnel.

**6.** Procédé selon l'une quelconque des revendications 1 à 5, **caractérisé en ce qu'**on utilise comme catalyseur de la triméthylamine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9828253 A
- WO 0147853 A
- EP 44444 A
- DE 1957591 A
- WO 9944974 A
- WO 9532171 A
- DE 1957591
- DE 1941633 A
- DE 2040501 A
- DE 2813201 A
- DE 2702582 A


### In der Beschreibung aufgeführte Nicht-Patentliteratur


- Ullmanns Encyklopädie der technischen Chemie. Verlag Chemie, 1972, vol. 2, 560-565